Europäisches Patentamt

(19) European Patent Office  (11) Publication number: **0 051 609**

Office européen des brevets  **B1**

(12) **EUROPEAN PATENT SPECIFICATION**

(45) Date of publication of patent specification: **28.11.84**  (51) Int. Cl.³: **A 61 B 17/30,** B 25 B 9/02, A 45 D 26/00

(21) Application number: **81901119.8**

(22) Date of filing: **29.04.81**

(86) International application number:
**PCT/SE81/00132**

(87) International publication number:
**WO 81/03121 12.11.81 Gazette 81/27**

(54) **TORSION RESISTANT FORCEPS.**

(30) Priority: **05.05.80 SE 8003353**

(43) Date of publication of application:
**19.05.82 Bulletin 82/20**

(45) Publication of the grant of the patent:
**28.11.84 Bulletin 84/48**

(84) Designated Contracting States:
**AT CH DE FR GB LI LU NL SE**

(56) References cited:
**GB-A-1 263 815**
**GB-A-2 035 187**
**SE-B- 388 770**
**US-A-3 648 702**
**US-A-4 044 771**

(73) Proprietor: **WANNAG, Arne**
**Palnäsvägen 11 A**
**S-133 00 Saltsjöbaden (SE)**

(72) Inventor: **WANNAG, Arne**
**Palnäsvägen 11 A**
**S-133 00 Saltsjöbaden (SE)**

(74) Representative: **Schöld, Zaid**
**c/o KemaNobel AB Patents Box 11065**
**S-100 61 Stockholm (SE)**

Courier Press, Leamington Spa, England.

## Description

### Technical field

The present invention relates to a forceps with a torsion resistant design. Especially the invention relates to disposable or reusable such a forceps made in plastic material.

### Background of the invention

A problem in connection with the design of forceps is that these must have a good lateral stability in order to avoid that the arms of the forceps at gripping pass each other like a pair of scissors, so that the grip is lost. These problems increase, when a finger grip and a thinner tip are tried to obtain. The problems also increase, when metal is abandoned as construction material and less rigid material like plastics is used.

Efforts have been made to cope with these problems by arranging different kinds of guiding pins on the interior sides of the forceps arms in order to avoid lateral displacement of the arms in relation to each other. These types of constructions, however, give bad lateral stability before the guiding mechanism has come into action, which means, that a lateral displacement can occur at the beginning of the movement, so that the guiding mechanism does not work at all. Further problems arise at gripping of larger object and it happens, that mops of e.g. cotton get stuck to the guide pins. Examples of constructions of this kind are disclosed in the US patent specifications 2.082.062, 3.140.715, 3.265.068, 3.367.336, 3.653.389 and 4.044.771, and GB—A—7263815.

### General description of the invention

The object of the present invention is to offer a forceps construction, which presents a very good lateral stability and torsion resistance without occurrence of the abovementioned problems. Another object of the invention is to offer a construction suitable for simple manufacturing in plastic materials. A further object of the invention is to offer a forceps with numerous fields of application.

These objects are reached with a forceps, having the characteristics of the appended claims.

In the usual manner the forceps has two arms, which are movably joined at a connection. According to the invention the arms have a wide base at the connection line, whereby an increased stability is obtained against lateral displacements of the arms. A further improvement of the stability is obtained, if the front parts of the arms have a design rigid to bending, while the rear parts, positioned at the joint, have a flexible design. If furthermore the rigid part of the arms has a substantially straight design, the risk is minimized for small deflections in load to cause rotational moments crossing the arms past each other. Further improvements of the stability are obtained, if the flexible rear parts of the arms are kept short in relation to the rigid front parts and if the joint area for the arms not only is wide but also has an essential thickness. Further advantages of the invention will be apparent from the detailed description below.

### Detailed description of the invention

According to the invention the arms shall have a wide base at the connection between these. A suitable ratio between the length of the arms and the width of the base is between 7:1 and 2:1. Preferably the ratio is between 6:1 and 2:1 and most preferably between 5:1 and 3:1. Such a design means that the arms cannot easily move laterally in relation to each other.

It is also desirable that the point of connection is designed in such a way that an essential rigidity against rotation is obtained. This can be achieved by giving the connection area not only a large width but also a substantial thickness, preferably being, at least at one part, thicker than the added thicknesses of the arms at the connection. This is preferably achieved by a separation of the arms from each other at the connection via a rounded off or flat bottom between the arms. The resulting accumulation of material gives an increased torsion resistance, which prevents the connection area from bending at rotation of one arm, so that the arms are mutually deflected.

The arms are suitably divided in a, in respect of bending, rigid front part and a flexible rear part close to the connection. The purpose thereof is, that all of the elastic bending, when pressing the arms together, substantially shall take place in the material in the rear flexible parts of the arms, while the remaining parts of the arms maintain their principle shape. As the flexible parts are found at the wide and torsion resistant part of the construction, the lateral rigidity can be maintained in spite of the flexibility. The flexible part shall, however, be kept as short as possible, and rather be made thin than long in order to maximize the lateral rigidity. Hence, it is preferred, that the length of the flexible part is less than 1:10 of the length of the rigid front part of the arms, and most preferably less than 1:12 of this part. A suitable profile of the flexible part is rectangular.

The part of the arms, rigid to bending, shall, as said, constitute the major part of the arms. The bending resistance can be obtained in several well-known ways but preferably the rigidity is obtained by arranging beams at the interior side of the arms, especially so that the arm profiles hereby become T-shaped. The rigid part of the arms shall be as straight as possible in design when viewed from the side. An arched design increases the torsional moment on the arms at small deflections in load, at the increased risk of crossing the arms. Suitably, the stiffening extends from the flexible rear part of the arms up to the tip area, which can, in respect of the intended use, have a separate design. Nor is it necessary to make the tip area straight, provided that this constitutes a minor part of the arm length. As a result of the torsion resistant design the tips can advantageously be made narrow, and it is preferred, that the arms as a whole have a shape, when vertically looked upon, mainly corresponding to an isosceles triangle. The interior sides of the tips should be slightly bevelled, so that the forceps begins its gripping at its outermost part. Behind the tip area the inner sides of the arms can be provided with coarse serrations, preferably by means of grooves in the stiffening beams, whereby the grasping of larger objects by using these parts of the forceps will be possible, e.g. plastic tubes at catheterization. This will considerably increase the field of application of the forceps.

The forceps can be manufactured in metal but preferably they are made of synthetic materials and especially then plastic materials like polyethene. Polypropene mixing with glass fibres has turned out to be a very suitable material. Injection moulding is a preferred manufacturing method.

Short description of the drawings

The figure sheet shows a preferred embodiment of the forceps of the invention. Figure 1 shows the forceps in an open position in side view. Figure 2 shows the forceps in a closed position also in side view. Figure 3 shows the forceps in plan view.

Description of the drawings

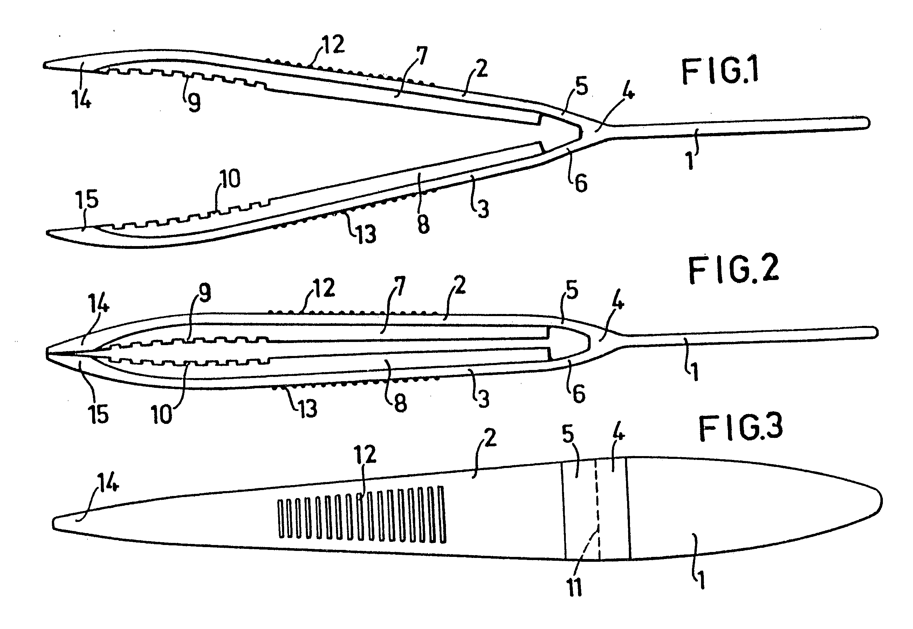

Position 1 indicates a flat handle part, intended to give the forceps a more applicable form for holding. The two arms 2 and 3, respectively, are connected at 4, where the material thickness has been increased, forming a flat portion between the arms at the connection line. Closest to connection 4, the arms consist of flexible parts 5 and 6, respectively, with principally a rectangular cross-section. The remaining parts of the arms are rigid, in respect of bending, through the presence of stiffening beams 7 and 8 on the inner sides of the arms. The front parts of the beams are equipped with coarse serrations 9 and 10, respectively, suitable for grasping of e.g. plastic tubes. Figure 3 shows the wide design of the forceps at the connection point 11 for the arms. On the exterior sides of the arms there are serrations 12 and 13, respectively, allowing a safe grip. The tips 14 and 15, respectivley, of the arms are designed with a flat surface along a part of the arm lengths, and the beams 7 and 8 form a continuation of these flat surfaces. As best can be seen from figure 2 the tip surfaces are bevelled, so that the outermost parts of the tips firstly meet at the compression of the arms. From figure 3 can be seen, that the tips are narrow and that the arms have the principal shape of an isosceles triangle, which, however, more rapidly tapers off towards the tip. From figures 1 and 2 can be seen, that the arms 2 and 3 principally are of a straight design between the flexible parts 5 and 6, respectively, and the tips 14 and 15, respectively, close to which the arms arch to form a thinner tip. When pressing the arms 2 and 3 together, these will substantially remain straight as a result of their rigid design, while the flexure takes place at the flexible rear parts 5 and 6. The short parts 5 and 6, the rigid connection 4, the wide base 11 of the arms and their straight and, in respect of bending, rigid design are features giving excellent torsion rigidity and lateral stability to the arms with a very small risk of crossing the arms past each other when gripping.

Claims

1. Forceps comprising two arms (2, 3) with tips (14, 15), rigid front parts, flexible rear parts (5, 6), and a base (4) connecting the arms adjacent the rear parts, wherein the arms have substantially the shape of an isosceles triangle in plan view, characterized in that, the ratio between the length of the arms (2, 3) and their width at the base in plan view is between 7:1 and 2:1.

2. The forceps of claim 1, characterized in, that the ratio is between 5:1 and 3:1.

3. The forceps of claim 1, characterized in, that the front parts of the arms (2, 3) are made resistant to bending by arranging stiffening beams (7, 8) along the interior sides of the arms up to the tips (14, 15).

4. The forceps of claim 3, characterized in, that the beams (7, 8) principally give a T-shaped cross-section to the arms.

5. The forceps of claim 1, characterized in, that the interior sides of the arms (2, 3) have serrations (9, 10) at a distance from the tips (14, 15).

6. The forceps of claim 1 characterized in, that the length of the flexible rear parts (5, 6) is shorter than 1:10 of the length of the rigid front parts of the arms (2, 3).

7. The forceps of claim 6, characterized in, that the ratio is less than 1:12.

**Patentansprüche**

1. Pinzette umfassend zwei Arme (2, 3) mit Spitzen (14, 15), festen vorderen Teilen, flexiblen hinteren Teilen (5, 6) und einer Basis (4), die die Arme neben den hinteren Teilen verbindet, wobei die Arme im wesentlichen die Form eines gleichschenkligen Dreickes in einer Draufsicht haben, dadurch gekennzeichnet, daß das Verhältnis zwischen der Länge der Arme (2, 3) und ihrer Breite an der Basis in der Draufsicht zwischen 7:1 und 2:1 leigt.

2. Pinzette nach Anspruch 1, dadurch gekennzeichnet, daß das Verhältnis zwischen 5:1 und 3:1 liegt.

3. Pinzette nach Anspruch 1, dadurch gekennzeichnet, daß die vorderen Teile der Arme (2, 3) dadurch biegefest gemacht sind, daß Versteifungsstege (7, 8) längs der Innenseiten der Arme bis zu den Spitzen (14, 15) angeordnet sind.

4. Pinzette nach Anspruch 3, dadurch gekennzeichnet, daß die Stege (7) und (8) den Armen einen im wesentlichen T-förmigen Querschnitt geben.

5. Pinzette nach Anspruch 1, dadurch gekennzeichnet, daß die Innenseiten der Arme (2, 3) Riffelungen (9, 10) in einem Abstand von den Spitzen (14, 15) haben.

6. Pinzette nach Anspruch 1, dadurch gekennzeichnet, daß die Länge der flexiblen hinteren Teile (5, 6) kleiner als 1:10 der Länge der festen vorderen Teile der Arme (2, 3) ist.

7. Pinzette nach Anspruch 6, dadurch gekennzeichnet, daß das Verhältnis kleiner als 1:12 ist.

## Revendications

1. Pinces comprenant deux branches (2, 3) avec des points (14, 15), des parties frontales rigides, des parties postérieures flexibles (5, 6), et une base (4) assurant la connexion des branches près des parties postérieures, dans lesquelles les branches ont, en plan, sensiblement la forme d'un triangle isocèle, caractérisées en ce que le rapport entre la longueur des branches (2, 3) et leur largeur à la base, vues en plan, est compris entre 7:1 et 2:1.

2. Pinces selon la revendication 1, caractérisées en ce que ledit rapport est compris entre 5:1 et 3:1.

3. Pinces selon la revendication 1, caractérisée en ce que les parties frontales des branches (2, 3) sont rendues résistantes à la torsion en prévoyant des armatures de raidissement (7, 8) le long des côtés intérieurs des branches jusqu'aux pointes (14, 15).

4. Pinces selon la revendication 3, caractérisées en ce que les armatures de renforcement (7, 8) donnent principalement une section droite en forme de T aux branches.

5. Pinces selon la revendication 1, caractérisées en ce que les côtés internes des branches (2, 3) comportent des stries (9, 10) à une certaines distance des pointes (14, 15).

6. Pinces selon la revendication 1, caractérisées en ce que la longueur des parties postérieures flexibles (5, 6) est inférieure au 1:10 de la longueur des parties frontales rigides des branches (2, 3).

7. Pinces selon la revendication 6, caractérisées en ce que ledit rapport est inférieur à 1:12.

FIG.1  FIG.2  FIG.3